# EUROPEAN PATENT APPLICATION

(11) **EP 4 570 918 A1**
(43) Date of publication of application: **18.06.2025**
(21) Application number: 22954286.5
(22) Date of filing: 09.08.2022
(51) Int. Cl.: C12Q 1/66, C07D 487/04, G01N 21/64

(54) **DUAL-LUCIFERASE REPORTER GENE DETECTION SYSTEM AND USE THEREOF**

(71) Applicant: BGI Shenzhen, Shenzhen, Guangdong 518083 (CN)
(72) Inventor: ZHUO, Shitian, Shenzhen, Guangdong 518083 (CN); TENG, Bo, Shenzhen, Guangdong 518083 (CN); ZHANG, Wenwei, Shenzhen, Guangdong 518083 (CN); CHEN, Ao, Shenzhen, Guangdong 518083 (CN); XU, Xun, Shenzhen, Guangdong 518083 (CN)
(74) Representative: Ström & Gulliksson AB
(86) International application number: PCT/CN2022/111058
(87) International publication number: WO 2024/031306

(57) **Abstract**

Provided are a dual-luciferase reporter gene detection system and use thereof. The detection system includes Gaussia luciferase, Pleuromamma xiphias luciferase, and a substrate. The substrate is coelenterazine or a derivative thereof. Compared with other dual-luciferase reporter gene detection systems, the present dual-luciferase reporter gene detection system produces stronger signals and requires simple reaction conditions.

## Description

### FIELD

The present disclosure relates to the technical field of molecular biology, and particularly, to a dual-luciferase reporter gene detection system and use thereof.

### BACKGROUND

Luciferase is a general term for enzymes capable of producing biological fluorescence in the nature. Luciferase reporter assay refers to a detection system for detecting luciferase activity by using fluorescein as a substrate, and it is widely used in cell imaging, amino acid labeling, protein labeling and localization, specific recognition of antibodies, nucleic acid labeling, construction of dual reporter genes and gene sequencing.

Coelenterazine is the most abundant natural luciferase in nature. Coelenterazine can be used as the substrate of many luciferases, such as Renilla luciferase, Gaussia secretory luciferase, and jellyfish luminescent protein. Unlike the beetle fluorescein/luciferase system, the coelenterazine/luciferase system does not require adenosine triphosphate (ATP), which is more convenient for *in vivo* biological fluorescence research, and has higher luminous brightness. Therefore, the coelenterazine is often used as a luminescent substrate for reporter gene detection and living animal detection based on fluorescence analysis.

Different coelenterazine derivatives are different in terms of luminescence wavelength, cell membrane permeability, and light quantum efficiency, thereby exhibiting different experimental effects in the same application scenario. The differences in the luminescence spectral characteristics and substrates of different luciferases provide theoretical basis for constructing a dual-luciferase luminescent system with low cross-interference and high specificity.

Dual-luciferase luminescence system is widely used in the fields of gene sequencing, construction of dual reporter genes, and protein localization. Such a method has the advantages such as high sensitivity, low interference, convenient detection, and wide detection range. Compared with single luciferase detection, multiple-detection is more suitable for: simultaneously studying the expression regulation of multiple genes; reducing the off-target effect; identifying the interaction between two or more signal paths; and normalizing the "artifact" generated by the experimental system.

At present, the existing dominant dual-luciferase luminescence system is a two-color detection composed of Firefly luciferase and Renilla luciferase. Firefly luciferase can only emit light when fluorescein, oxygen, ATP and magnesium ions are present at the same time. However, the luminescence of Renilla luciferase only requires the presence of coelenterazine and oxygen. The difference in reaction conditions between these two luciferase systems limits their application in the same scenario.

### SUMMARY

In view of the above, the present disclosure provides a dual-luciferase reporter gene detection system and the use thereof.

The present disclosure provides a dual-luciferase reporter gene detection system, including Gaussia luciferase, Pleuromamma xiphias luciferase, and a substrate. The substrate is a compound represented by formula (I), or a stereoisomer, geometric isomer, tautomer, salt, nitrogen oxide, hydrate or solvate of the compound represented by formula (I): 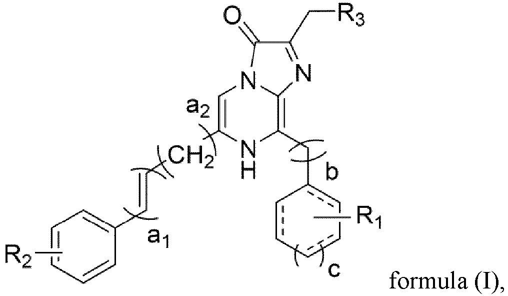 where:
R₁ and R₂ are each independently selected from H, D, F, Cl, Br, I, OH, NH₂, NO₂, CN, N₃, C₁₋₆ alkyl, C₂₋₆ alkenyl, C₂₋₆ alkynyl, halogenated C₁₋₆ alkyl, C₁₋₆ alkoxy, C₁₋₆ hydroxyalkyl, or C₁₋₆ alkylamino;
R₃ is an optionally substituted aryl, heterocyclic group or heteroaryl;
a₁ is 0 or any integer ranging from 1 to 6,
a₂ is 0 or any integer ranging from 1 to 6;
b is 0 or 1,
C is 0, 1 or 2.

In the present disclosure, the Gaussia luciferase is any one of I) to III):
I) Gaussia luciferase having an amino acid sequence as set forth in SEQ ID NO: 1;
II) luciferase having at least 85% homology with the amino acid sequence of the Gaussia luciferase as defined in I) and having the same or similar function as the Gaussia luciferase as defined in I);
III) luciferase having an amino acid sequence of the Gaussia luciferase as defined in I) in which one or more amino acid residues are modified, substituted, deleted or added and having Gaussia luciferase activity.

In some embodiments, the Gaussia luciferase is not subjected to optimization and derived from *Gaussia luciferase,* and its amino acid sequence is the sequence of the GenBank accession number of AY015993.1 (as set forth in SEQ ID NO:1).

In some embodiments, the Gaussia luciferase is the luciferase having at least 85%, for example, at least 86%, 87%, 88%, 89%, 90%, 91%, 92%, 93%, 94%, 95%, 96%, 97%, 98% or 99% homology with the amino acid sequence of the Gaussia luciferase as defined in I) and having the same or similar function as the Gaussia luciferase as defined in I).

In some embodiments, the Gaussia luciferase is an optimized mutant, i.e., Gaussia luciferase mutant, and has a mutation at least one of following mutation sites in the amino acid sequence as set forth in SEQ ID NO: 1: sites 24, 26, 27, 29, 30, 31, 32, 33, 36, 37, 40, 66, 79, 84, 88, 102, 103, 104, 110, 123, 124, 138, 152, 163, 167, 170, 174, 175, 178, 182 and 183.

In some embodiments, the Gaussia luciferase mutant has at least one of the following mutations:
1) E at site 24 being mutated to K;
2) F at site 26 being mutated to R or L;
3) N at site 27 being mutated to D;
4) V at site 29 being mutated to F or L;
5) A at site 30 being mutated to G or D;
6) V at site 31 being mutated to I;
7) A at site 32 being mutated to V;
8) S at site 33 being mutated to E, R, or K;
9) A at site 36 being mutated to V or I;
10) T at site 37 being mutated to N or E;
11) L at site 40 being mutated to I or T;
12) K at site 66 being mutated to P, S, I, R, or N;
13) H at site 79 being mutated to K;
14) P at site 84 being mutated to A, L, K, or V;
15) K at site 88 being mutated to R;
16) E at site 102 being mutated to D, A, S, K, or N;
17) S at site 103 being mutated to T;
18) A at site 104 being mutated to G;
19) E at site 110 being mutated to P, G, or A;
20) D at site 123 being mutated to N;
21) L at site 124 being mutated to M, G or I;
22) V at site 138 being mutated to E or D;
23) Q at site 152 being mutated to R or H;
24) Q at site 163 being mutated to D;
25) S at site 170 being mutated to N or T;
26) G at site 174 being mutated to K;
27) Q at site 175 being mutated to E;
28) K at site 178 being mutated to T;
29) A at site 182 being mutated to M;
30) G at site 183 being mutated to N or A.

Compared with the wild-type Gaussia luciferase, such a mutant has a wider substrate spectrum, higher specificity, and significantly enhanced luminous brightness. Thus, its detection accuracy in practical application is significantly improved.

In some embodiments, the Gaussia luciferase may or may not include an amino acid sequence of a signal peptide.

In the present disclosure, the Pleuromamma xiphias luciferase is any one of I) to III):
I) Pleuromamma xiphias luciferase having an amino acid sequence as set forth in SEQ ID NO: 3;
II) luciferase having at least 85% homology with the amino acid sequence of the Pleuromamma xiphias luciferase as defined in I) and having the same or similar function as the Pleuromamma xiphias luciferase as defined in I);
III) luciferase having an amino acid sequence of the Pleuromamma xiphias luciferase as defined in I) in which one or more amino acid residues are modified, substituted, deleted or added and having Pleuromamma xiphias luciferase activity.

In some embodiments, the Pleuromamma xiphias luciferase is not optimized and derived from *Pleuromamma xiphias,* and its amino acid sequence is the sequence with PMID number of 23886588 (as set forth in SEQ ID NO: 3).

In some embodiments, the Pleuromamma xiphias luciferase is the luciferase having at least 85%, for example at least 86%, 87%, 88%, 89%, 90%, 91%, 92%, 93%, 94%, 95%, 96%, 97%, 98%, or 99% homology with the amino acid sequence of the Pleuromamma xiphias luciferase as defined in I) and having the same or similar function as the Pleuromamma xiphias luciferase as defined in I).

In some embodiments, the Pleuromamma xiphias luciferase is an optimized mutant, i.e., Pleuromamma xiphias luciferase mutant, and has a mutation at least one of following mutation sites in the amino acid sequence as set forth in SEQ ID NO: 3: sites 81, 82, 83 and 84.

In some embodiments, the Pleuromamma xiphias luciferase mutant has at least one of the following mutations:
(1) G at site 81 being mutated to L, P, Q, S, or T;
(2) Q at site 82 being mutated to R, W, I, Y, A, L, F, V, P, E, or M;
(3) G at site 83 being mutated to S, Q, R, W, T, A, or L;
(4) G at site 84 being mutated to F, R, S, C, Y, L, I, K, V, or P.

Compared with the wild-type Pleuromamma xiphias luciferase, such a mutant has a wider substrate spectrum, higher specificity, and significantly enhanced luminous brightness. Thus, its detection accuracy in practical application is significantly improved.

In some embodiments, the Pleuromamma xiphias luciferase may or may not include an amino acid sequence of a signal peptide.

In some embodiments, the Pleuromamma xiphias luciferase includes an amino acid sequence of a signal peptide, and preferably, the amino acid sequence of the Pleuromamma xiphias luciferase including the signal peptide is as set forth in SEQ ID NO: 4.

In the embodiments of the present disclosure, the compound represented by formula (I) may further include at least one of the following additional technical features.

In some embodiments, R₁ is H.

In some specific embodiments, R₂ is H, NH₂, OH, or C₁₋₆ alkylamino, and preferably H, NH₂, 3-OH, 4-OH, or dimethylamino.

In some embodiments, R₃ is a substituted aryl, preferably OH-C₆H₅, or F-C₆H₅, and more preferably 4-OH-C₆H₅ or 4-F-C₆H₅.

In some embodiments, a₁ is 0 or 1.

In some embodiments, a₂ is 0 or 1.

In some embodiments, when a₁ is 0, a₂ is 1; when a₁ is 1, a₂ is 0; or when a₁ is 0, a₂ is 0.

In some embodiments, b is 1.

In some embodiments, c is 1.

In some embodiments, the compound represented by formula (I) includes any two of the following compounds: 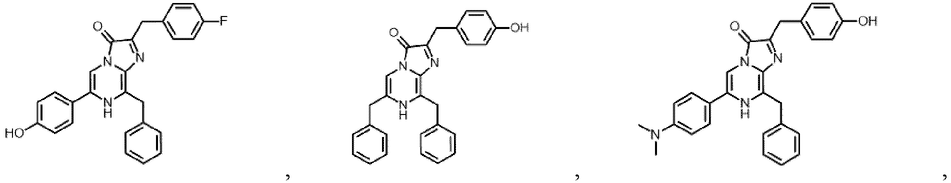 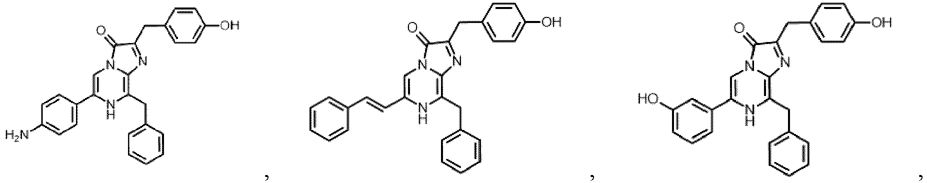 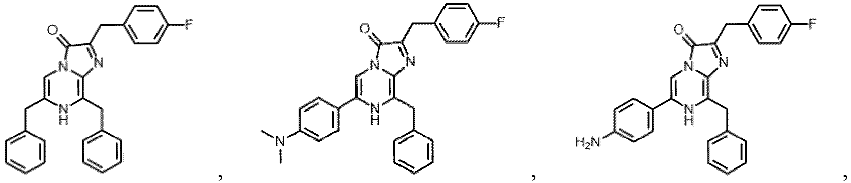 and

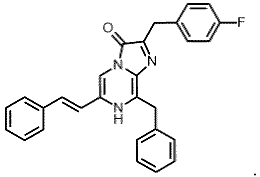

In some embodiments, a substrate of the Gaussia luciferase is

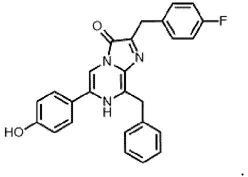

In some embodiments, a substrate of the Pleuromamma xiphias luciferase is

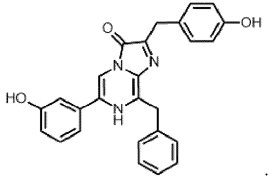

In some embodiments, a molar ratio of the Gaussia luciferase to the Pleuromamma xiphias luciferase is 1: (0.01 to 100).

In some embodiments, a molar ratio of the Gaussia luciferase to the substrate is 1: (1 to 1000).

In some embodiments, a molar ratio of the Pleuromamma xiphias luciferase to the substrate is 1: (1 to 1000).

The present disclosure further provides use of the dual-luciferase reporter gene detection system according to the present disclosure in the preparation of a detection reagent.

In the present disclosure, the detection reagent includes a cell imaging reagent, an amino acid labeling reagent, a protein labeling and localization reagent, an antibody specific recognition reagent, a nucleic acid labeling reagent, or a gene sequencing reagent.

The present disclosure further provides a fluorescence detection kit including the dual-luciferase reporter gene detection system according to the present disclosure.

In the present disclosure, the fluorescence detection reagent further includes a reaction buffer, and the reaction buffer includes water, Tris-HCl, NaCl, and Tween-20.

The present disclosure further provides a method for detecting a luminescent signal of luciferase. The method includes: contacting the substrate in the dual-luciferase reporter gene detection system as described in the present disclosure with a sample, and detecting luminescent signals generated by the Gaussia luciferase and the Pleuromamma xiphias luciferase. The sample contains Gaussia luciferase and Pleuromamma xiphias luciferase.

In the present disclosure, the sample is a nucleic acid, a nucleic acid analogue, a cell, a culture containing the cell or a product prepared from the culture containing the cell. The cell is a bacterial cell, fungal cell, animal cell, plant cell, or human cell. The nucleic acid is DNA or RNA, which is not limited in the present disclosure.

In some embodiments, the sample is a cell, a culture containing a cell, or a lysate of a cell, the cell expressing the Gaussia luciferase and the Pleuromamma xiphias luciferase in the dual-luciferase reporter gene detection system as described in the present disclosure.

In other embodiments, the sample is a nucleotide analogue capable of specifically binding to the Gaussia luciferase and the Pleuromamma xiphias luciferase in the dual-luciferase reporter gene detection system according to the present disclosure, and the nucleotide analogue specifically binds to the luciferase through a nucleophile.

The nucleophile is an antigen-antibody complex, or a biotin-streptavidin or a digoxin-digoxin antibody.

The nucleotide analogue consists of nucleotide molecules containing four different types of bases. The first nucleotide molecule is capable of specifically binding to the Gaussia luciferase, the second nucleotide molecule is capable of specifically binding to the Pleuromama Xiphias luciferase, the third nucleotide molecule is capable of specifically binding to the Gaussia luciferase and the Pleuromama Xiphias luciferase, and the fourth nucleotide molecule neither binds to the Gaussia luciferase nor the Pleuromamma xiphias luciferase.

In the present disclosure, the method further includes: determining a type of the nucleotide molecule based on the luminescent signal.

Further, the present disclosure further provides a method for sequencing a nucleic acid molecule, including:
(1) providing a to-be-sequenced nucleic acid molecule connected to a support, or connecting a to-be-sequenced nucleic acid molecule onto a support;
(2) forming a reaction system containing a solution phase and a solid phase by adding a primer for initiating a nucleotide polymerization reaction, a polymerase for performing the nucleotide polymerization reaction, and four compounds, where the four compounds are derivatives of nucleotides A, (T/U), C and G, respectively and have an ability of base complementary pairing, and where a hydroxyl group (-OH) at 3' site of ribose or deoxyribose of the four compounds is protected by a protecting group,
   a first compound is linked with a first molecular marker,
   a second compound is linked with a second molecular marker,
   a third compound is linked with the first molecular marker and the second molecular marker, or a part of the third compound is linked with the first molecular marker and the other part of the third compound is linked with the second molecular marker, and
   a fourth compound is not linked with a molecular marker;
(3) annealing the primer onto the to-be-sequenced nucleic acid molecule, where the primer, as an initial growing nucleic acid chain, forms a duplex connected to the support together with the to-be-sequenced nucleic acid molecule;
(4) performing the nucleotide polymerization reaction using the polymerase under the conditions of allowing the polymerase to perform a nucleotide polymerization reaction, to incorporate one of the four compounds to the 3' end of the growing nucleic acid chain;
(5) contacting the duplex in the previous step with the Gaussia luciferase and the Pleuromamma xiphias luciferase in the dual-luciferase reporter gene detection system of the present disclosure for a binding reaction, where the Gaussia luciferase and the Pleuromamma xiphias luciferase are capable of specifically binding to the first molecular marker and the second molecular marker, respectively, enabling the luciferase to undergo fluorescence reaction in the presence of a substrate, and detecting emitted fluorescence signal;
(6) removing the protective groups and molecular markers of the incorporated nucleotides;
(7) optionally repeating steps (4) to (6) or steps (3) to (6) for one or more times, to obtain the sequence information of the nucleic acid molecule.

In the embodiments of the present disclosure, by connecting luciferase and nucleotide derivative, a self-luminescence detection of nucleotides to be sequenced can realized without requiring an additional excitation light source. In a specific embodiment, luciferase and nucleotide can be connected to each other by specifically binding a label on the luciferase to a corresponding label on the nucleotide derivative. In a specific embodiment, the first nucleotide is linked with the first luciferase, the second nucleotide is linked with the second luciferase, the third nucleotide is linked with the first luciferase and the second luciferase, and the fourth nucleotide is not linked with any luciferase. Then, the corresponding substrates of these two luciferases are respectively introduced to detect the luminescence signals of four bases. When the substrate of the first luciferase is introduced, the first nucleotide and the third nucleotide emit light; when the substrate of the second luciferase is introduced, the second nucleotide and the third nucleotide emit light; and when the substrates of the first luciferase and the second luciferase are both introduced, the fourth nucleotide does not emit light. Therefore, the base can be identified based on the light-emitting conditions of the four nucleotides.

Further, the method for sequencing the nucleic acid molecules according to the present disclosure includes:
(1) providing a to-be-sequenced nucleic acid molecule connected to a support, or connecting the to-be-sequenced nucleic acid molecule to a support;
(2) forming a reaction system containing a solution phase and a solid phase by adding a primer for initiating a nucleotide polymerization reaction, a polymerase for performing the nucleotide polymerization reaction, and four compounds, where the four compounds are derivatives of nucleotides A, (T/U), C and G, respectively and have an ability of base complementary pairing, and where a hydroxyl group (-OH) at 3' site of ribose or deoxyribose of the four compounds is protected by a protecting group,
   a first compound is linked with a first molecular marker,
   a second compound is linked with a second molecular marker,
   a third compound is linked with a first molecular marker and a second molecular marker, or a part of the third compound is linked with a first molecular marker and the other part of the third compound is linked with a second molecular marker, and
   a fourth compound is not linked with a molecular marker;
(3) annealing the primer onto the to-be-sequenced nucleic acid molecule, where the primer, as an initial growing nucleic acid chain, forms a duplex connected to the support together with the to-be-sequenced nucleic acid molecule;
(4) performing the nucleotide polymerization reaction using the polymerase under the conditions of allowing the polymerase to perform a nucleotide polymerization reaction, to incorporate one of the four compounds into the 3' end of the growing nucleic acid chain;
(5) removing the solution phase of the reaction system in the previous step, keeping the duplex connected to the support, and adding two different luciferases for binding reaction, where the two luciferases are capable of specifically binding to the first molecular marker and the second molecular marker, respectively;
(6) removing the unbound luciferase with an elution buffer;
(7) adding a substrate of the first luciferase to detect a first fluorescence signal;
(8) removing the solution of the previous step;
(9) adding a substrate of the second luciferase to detect a second fluorescence signal;
(10) removing the solution of the previous step;
(11) removing the protective groups and molecular markers of the incorporated nucleotides;
(12) optionally, removing the solution of the previous step;
(13) optionally, repeating steps (3) to (12) or steps (4) to (11) for one or more times, to obtain the sequence information of the nucleic acid molecule.

In the present disclosure, wild-type/mutant *Gaussia luciferase* and wild-type/mutant Pleuromamma xiphias luciferase (Ab716975) are combined. By screening the luminescent effects of different coelenterazine derivatives with Gaussia luciferase and Pleuromamma xiphias luciferase, a dual-luciferase reporter gene detection system with low cross-interference is obtained. Compared with the combinations of other dual-luciferase, the reaction conditions of the present dual-luciferase combination are simple, only requiring coelenterazine and oxygen, and the operation is simpler.

### DETAILED DESCRIPTION

The present disclosure discloses a dual-luciferase reporter gene detection system and a use thereof, and those skilled in the art can learn from the contents herein and improve the process parameters appropriately. It should be particularly pointed out that all similar alternatives and modifications will be apparent to those skilled in the art, and they are all considered to be included in the present disclosure. The method and use of the present disclosure have been described through preferred embodiments. In order to realize and apply the technology of the present disclosure, those skilled in the art can obviously modify or appropriately change and combine the method and use described herein without departing from the content, spirit and scope of the present disclosure.

### Definitions and General terms

In the description of the present disclosure, unless otherwise specified, "a plurality of" means two or more.

In the present disclosure, the documents corresponding to the specific contents will be listed in detail, and the examples are accompanied by diagrams of structural formulas and chemical formulas. The present disclosure is intended to cover all alternatives, modifications and equivalents, which may be included in the existing field of present disclosure as defined by the claims. Those skilled in the art will recognize many methods and substances similar to or equivalent to those described herein, which can be applied to the practice of the present disclosure. The present disclosure is by no means limited to the description of methods and substances. There are many documents and similar substances that are different from or conflict with the present disclosure, including but by no means limited to the definitions of terms, the usage of terms, the techniques described, or the scope controlled by the present disclosure.

The present disclosure will apply the following definitions unless otherwise indicated. According to the purpose of the present disclosure, the chemical elements are defined according to the periodic table of elements, CAS versions and Handbook of Chemicals, 75, thEd, 1994. In addition, the general principles of organic chemistry can be found in "Organic Chemistry" Thomas Sorrell, University Science Books, Sausalito: 1999, and "March's Advanced Organic Chemistry" by Michael B. Smith and Jerry March, John Wiley & Sons, New York: 2007, so all the contents are integrated with references.

The term "comprise" or include" is an open-ended expression, i.e., including the items indicated in the present disclosure, but not excluding items of other aspects.

The compounds as described herein may be optionally substituted with one or more substituents, such as the compounds of the general formula according to the present disclosure, or the special examples, subclasses in the examples, and a class of compounds encompassed by the present disclosure. It should be understood that the term "optionally substituted" can be used interchangeably with the term "substituted or unsubstituted". Generally, the term "optionally", whether preceded by the term "substituted" or not, indicates that one or more hydrogen atoms in a given structure are substituted by specific substituents. Unless otherwise indicated, an optional substituent group can have one substituent that is substituted at any substitutable site of the group. When more than one site in the given structural formula can be substituted by one or more substituents selected from specific groups, the substituents can be substituted at respective sites equally or differently. The substituents can be, but are not limited to, hydrogen, F, Cl, Br, I, nitro, cyano, oxo (=O), hydroxyl, alkyl, hydroxyalkyl, alkylamino, aminoalkyl, haloalkoxy, cycloalkyl, amino, aryl, heterocyclyl, heteroaryl, alkenyl, alkynyl, cycloalkyloxy, alkoxy, alkoxyalkyl, haloalkyl, -COOH, -alkylene-C(=O)O-alkyl, -alkylene-S(=O)₂-alkyl, -alkylene-S(=O)₂-amino, -S(=O)₂-alkyl, -S(=O)₂-amino, -S(=O)₂OH, -O-alkylene-C(=O)O-alkyl, -O-alkylene-S(=O)₂-alkyl, -O-alkylene-S(=O)₂-amino, -O-alkylene-S(=O)₂OH, -C(=O)NH₂, -C(=O)NH-alkyl, -C(=O)N(alkyl)-alkyl, -C(=O)NHS(=O)₂-alkyl, -C(=O)NHS(=O)₂-amino, -C(=O)NHS(=O)₂OH, -N(haloalkyl)-alkyl, -N(alkyl)-S(=O)₂-alkyl, -NHS(=O)₂-alkyl, -NHS(=O)₂-haloalkyl, -N(alkyl)S(=O)₂-haloalkyl, -N(alkyl)S(=O)₂-alkylamino, -NHC(=O)-alkyl, -NHC(=O)-haloalkyl, -N(alkyl)C(=O)-haloalkyl, -N(alkyl)C(=O)-alkylamino, -N(alkyl)C(=O)O-alkyl, -NHC(=O)O-alkyl, -NHC(=O)O-haloalkyl, -N(alkyl)C(=O)O-haloalkyl, -N(alkyl)C(=O)O-aminoalkyl, -NHC(=O)-NH₂, -NHC(=O)NH-(alkyl), -NHC(=O)NH(haloalkyl), -NHC(=O)N(alkyl)-alkyl, -OC(=O)-alkyl, -OC(=O)-amino, -OC(=O)-alkylamino, -OC(=O)-aminoalkyl, -OC(=O)-alkoxy, -C(=O)N(alkyl)S(=O)₂-alkyl, -C(=O)N(alkyl)S(=O)₂-amino, -C(=O)NH-S(=O)₂OH, -C(=NH)NH₂, -C(=NH)NH-alkyl, -C(=NH)N(alkyl)-alkyl, -C(=N-alkyl)-NH₂, -C(=O)NH-alkylene-S(=O)₂OH, -C(=O)NHC(=O)OH, -C(=O)NHC(=O)O-alkyl, -C(=O)N(alkyl)C(=O)O-alkyl, -C(=O)NH-alkylene-C(=O)OH, and -C(=O)NH-alkylene-C(=O)O-alkyl, etc.

The term "alkyl" used in the present disclosure refers to a saturated linear or branched monovalent hydrocarbon radical including 1 to 20 carbon atoms, or 1 to 10 carbon atoms, or 1 to 6 carbon atoms, or 1 to 4 carbon atoms, or 1 to 3 carbon atoms, or 1 to 2 carbon atoms, in which alkyl may be independently and optionally substituted by one or more substituents as described in the present disclosure. Further examples of alkyl group include, but are not limited to, methyl (Me, -CH₃), ethyl (Et, -CH₂CH₃), n-propyl (*n*-Pr, -CH₂CH₂CH₃), isopropyl (*i*-Pr, -CH(CH₃)₂), n-butyl (*n*-Bu, -CH₂CH₂CH₂CH₃), isobutyl (*i*-Bu, -CH₂CH(CH₃)₂), sec-butyl (*s*-Bu, -CH(CH₃)CH₂CH₃), tert-butyl (*t*-Bu, -C(CH₃)₃), n-pentyl (-CH₂CH₂CH₂CH₂CH₃), 2-pentyl (-CH (CH₃)CH₂CH₂CH₃), 3-pentyl (-CH (CH₂CH₃)₂), 2-methyl-2-butyl (-C(CH₃)₂CH₂CH₃), 3-methyl-2-butyl (-CH(CH₃)CH(CH₃)₂), 3-methyl-1-butyl (-CH₂CH₂CH(CH₃)₂), 2-methyl-1-butyl (-CH₂CH(CH₃)CH₂CH₃), n-hexyl (-CH₂CH₂CH₂CH₂CH₂CH₃), 2-hexyl (-CH(CH₃)CH₂CH₂CH₂CH₃), 3-hexyl (-CH(CH₂CH₃) (CH₂CH₂CH₃)), 2-methyl-2-pentyl (-C(CH₃)₂CH₂CH₂CH₃), 3-methyl-2-pentyl (-CH(CH₃)CH(CH₃)CH₂CH₃), 4-methyl-2-pentyl (-CH(CH₃)CH₂CH (CH₃)₂), 3-methyl-3-pentyl (-C(CH₃)(CH₂CH₃)₂), 2-methyl-3-pentyl (-CH(CH₂CH₃)CH(CH₃)₂), 2,3-dimethyl-2-butyl (-C(CH₃)₂CH(CH₃)₂), 3,3-dimethyl-2-butyl (-CH(CH₃)C(CH₃)₃), n-heptyl, and n-octyl, etc. As used herein, the term "alkyl" and "alkane" both include linear and branched saturated carbon chains.

The term "alkenyl" refers to a linear and branched monovalent hydrocarbon radical having 2 to12 carbon atoms, or 2 to 8 carbon atoms, or 2 to 6 carbon atoms, or 2 to 4 carbon atoms, in which at least one site is unsaturated, i.e., one carbon-carbon is sp² double bond, and in which alkenyl may be independently and optionally substituted by one or more substituents as described in the present disclosure, including the groups with a "trans" "cis" or "E" "Z" orientation. Specific examples of alkenyl include, but are not limited to, vinyl (-CH=CH₂), allyl (-CH₂CH=CH₂), and alkenyl butyl (-CH₂CH₂CH=CH₂), etc.

The term "alkynyl" refers to a linear or branched monovalent hydrocarbon radical having 2 to 12 carbon atoms, or 2 to 8 carbon atoms, or 2 to 6 carbon atoms, or 2 to 4 carbon atoms, in which at least one site is unsaturated, i.e., one carbon-carbon is sp triple bond, and in which the alkynyl group may be independently and optionally substituted by one or more substituents described in the present disclosure. Specific examples include, but are not limited to, ethynyl (-C≡CH) and propargyl (-CH₂C≡CH).

The term "halogen" refers to F, Cl, Br, or I.

As used in the present disclosure, the term "unsaturated" means that the moiety contains one or more unsaturation.

As used in the present disclosure, the term "alkoxy" or "alkyloxy" refers to an alkyl group as defined in the present disclosure connected to other parts of the compound molecule through an oxygen atom. In some embodiments, alkoxy is C₁₋₄ alkoxy, and examples thereof include, but are not limited to, methoxy, ethoxy, propoxy, butoxy, and the like. Moreover, alkoxy may be independently unsubstituted or substituted by one or more substituents as described in the present disclosure.

As used in the present disclosure, the terms "haloalkyl", "haloalkenyl", and "haloalkoxy" respectively refer to alkyl, alkenyl, and alkyloxy substituted by one or more halogen atoms. In some embodiments, haloalkyl is halogenated C₁₋₆ alkyl. In other embodiments, haloalkyl is halogenated C₁₋₃ alkyl. In some embodiments, haloalkyloxy or haloalkoxy is halogenated C₁₋₆ alkyloxy or halogenated C₁₋₆ alkoxy. In other embodiments, haloalkyloxy or haloalkoxy is halogenated C₁₋₃ alkyloxy or halogenated C₁₋₃alkoxy, and examples thereof include, but are not limited to, trifluoromethyl, 2-chloro-vinyl, 2,2-difluoroethyl, trifluoromethoxy, and the like. The "haloalkyl", "haloalkenyl" and "haloalkyloxy" group may be independently and optionally substituted by one or more substituents as described in the present disclosure.

The term "cycloalkyl" or "cycloalkane" refers to a monovalent or polyvalent saturated monocyclic, bicyclic or tricyclic carbocyclic system containing 3 to 12 carbon atoms, but containing no aromatic ring. In an embodiment, cycloalkyl contains 3 to 12 carbon atoms. In another embodiment, cycloalkyl contains 3 to 8 carbon atoms. In yet another embodiment, cycloalkyl contains 3 to 6 carbon atoms. Examples thereof include, but are not limited to, cyclopropyl, cyclobutyl, cyclopentyl, cyclohexyl, and the like. Cycloalkyl may be independently unsubstituted or substituted by one or more substituents described in the present disclosure.

The terms "heterocyclyl" and "heterocyclic ring" are used interchangeably herein and both refer to a saturated or partially unsaturated monocyclic, bicyclic or tricyclic ring containing 3 to 12 ring atoms, but containing no aromatic ring, in which at least one ring atom is a heteroatom. Unless otherwise specified, heterocyclyl can be carbon-based or nitrogen-based, and the heteroatom has the meaning as described in the present disclosure. Examples of heterocyclyl include, but are not limited to, oxiranyl, azacyclobutyl, oxetanyl, thiacyclobutyl, pyrrolidinyl, 2-pyrrolinyl, 3-pyrrolinyl, pyrazolinyl, pyrazolidinyl imidazolinyl, imidazolidinyl, tetrahydrofuranyl, dihydrofuranyl, tetrahydrothienyl, dihydrothienyl, 1,3-dioxy-cyclopentyl dithiocyclopentyl, tetrahydropyranyl, dihydropyranyl, 2*H*-pyranyl, 4*H*-pyranyl, tetrahydropyranyl, piperidinyl, morpholinyl, thiomorpholinyl, piperazinyl, dioxanyl, dithianyl, thioxanyl, homopiperazinyl, homopiperidinyl, oxacyclohetyl, thiocyclohepty, oxazepinyl, diazepinyl, thiazepinyl, and 2-oxa-5-azabicyclo [2.2.1]heptyl-5-yl. Examples of heterocyclyl in which -CH₂- is substituted by -C(=O)- include, but are not limited to, 2-oxopyrrolidinyl, oxo-1,3-thiazolidinyl, 2-piperidonyl, 3,5-dioxopiperidinyl, and pyrimidinedionyl. Examples of heterocyclyl in which sulfur atom is oxidized include, but are not limited to, sulfolanyl and 1,1-dioxothiomorpholinyl. Heterocyclyl may be optionally substituted by one or more substituents as described in the present disclosure.

The term "heteroaryl" refers to a monocyclic, bicyclic and tricyclic system containing 5 to 12 ring atoms, or 5 to 10 ring atoms, or 5 to 6 ring atoms, in which at least one ring system is an aromatic ring and at least one ring system contains one or more heteroatoms, and in which each ring contains a ring consisting of 5 to 7 atoms and has one or more attachment points linked with the rest of the molecule. The term "heteroaryl" can be used interchangeably with the terms "heteroaromatic ring" or "heteroaromatic compound". Heteroaryl may be optionally substituted with one or more substituents as described in the present disclosure. In an embodiment, heteroaryl consisting of 5 to10 atoms contains 1, 2, 3 or 4 heteroatoms independently selected from O, S and N, in which the nitrogen atom can be further oxidized.

Examples of heteroaryl include, but not limited to: furyl, imidazolyl (such as N-imidazolyl, 2-imidazolyl, 4-imidazolyl, 5-imidazolyl), isoxazolyl, oxazolyl (such as 2-oxazolyl, 4-oxazolyl, 5-oxazolyl), pyrrolyl (such as N-pyrrolyl, 2-pyrrolyl, 3-pyrrolyl), pyridyl, pyrimidinyl (such as 2-pyrimidinyl, 4-pyrimidinyl, 5-pyrimidinyl), pyridazinyl, thiazolyl (such as 2-thiazolyl, 4-thiazolyl, 5-thiazolyl), tetrazolyl (such as 5-tetrazolyl), triazolyl, thienyl (such as 2-thienyl and 3-thienyl), pyrazolyl, isothiazolyl, 1,2,3-oxadiazolyl, 1,2,5-oxadiazolyl, 1,2,4-oxadiazolyl, 1,2,3-triazolyl, 1,2,3-thiodiazolyl, 1,3,4-thiodiazolyl, 1,2,5-thiodiazolyl, pyrazinyl, and 1,3,5- triazine. Examples of heteroaryl include, but not limited to, the following bicyclic rings: benzimidazolyl, benzofuranyl, benzothiophenyl, indolyl (such as 2-indolyl), purinyl, quinolyl (such as 2-quinolyl, 3-quinolyl, 4-quinolyl), 1,2,3,4-tetrahydroisoquinolyl, 1,3-benzodioxomoyl, indolinyl, isoquinolinyl (such as 1-isoquinolinyl, 3- isoquinolinyl or 4-isoquinolinyl), imidazo[1,2-*a*] pyridyl, pyrazolo[1,5-a] pyridyl, pyrazolo[1,5-a]pyrimidinyl, imidazo[1,2-*b*]pyridazinyl, [1,2,4]triazolo[4,3-*b*]pyridazinyl, [1,2,4]triazolo[1,5-*a*]pyrimidinyl, and [1,2,4]triazolo[1,5-*a*]pyridyl, and the like.

The term "aminoalkyl" refers to linear or branched C₁₋₁₀ alkyl substituted by one or more amino groups. In some embodiments, aminoalkyl is C₁₋₆ alkyl substituted by one or more amino groups, and examples thereof include, but are not limited to, aminomethyl, aminoethyl, aminopropyl, aminobutyl and aminohexyl. Aminoalkyl may optionally be substituted by one or more substituents described in the present disclosure.

The term "hydroxyalkyl" refers to alkyl substituted by one or more hydroxyl groups, in which alkyl is as defined in the present disclosure. Examples thereof include, but are not limited to, hydroxymethyl, hydroxyethyl and 1,2-dihydroxyethyl, and the like.

As described in the present disclosure, when a substituent is presented as being connected to the central ring through painted bond, it indicates that such a substituent can be substituted at any substitutable site on the ring. For example, Formula (a) represents one or more substituents R° can be substituted at any site on the ring E.

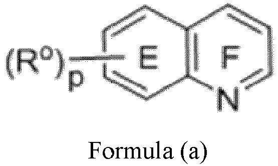

In addition, it should be noted that, unless otherwise explicitly pointed out, the descriptions in the manners of "each of ... and ...is independently" or "... and ... are each independently" used throughout the present disclosure are interchangeable, and should be understood in a broad sense. It can mean that the specific options expressed by the same symbol in different groups do not affect each other, or it can mean that the specific options expressed by the same symbol in the same group do not affect each other.

Unless otherwise indicated, the structural formula described in the present disclosure includes all isomeric forms (such as enantiomers, diastereomers, geometric isomers or conformational isomers): for example, R and S configurations containing asymmetric centers, (Z) and (E) isomers of double bonds, and (Z) and (E) conformational isomers. Therefore, individual stereochemical isomers of the compound of the present disclosure or a mixture of enantiomers, diastereomers, geometric isomers or conformational isomers thereof of the present disclosure belong to the scope of the present disclosure.

Unless otherwise indicated, the structural formulas and compounds described in the present disclosure include all isomeric forms (such as enantiomers, diastereomers, geometric isomers or conformational isomers), salts, nitrogen oxides, hydrates or solvates. Therefore, an individual stereochemical isomer, enantiomer, diastereomer, geometric isomer, conformational isomer, salt, nitrogen oxide, hydrate and solvate of the compound of the present disclosure also belong to the scope of the present disclosure. In addition, unless otherwise indicated, the structural formula of the compound described in the present disclosure includes enriched isotopes of one or more different atoms.

The definitions and conventions of stereochemistry in the present disclosure can generally refer to the following documents: S.P. Parker, Ed., McGraw-Hill Dictionary of Chemical Terminology (1984) McGraw-Hill Book Company, New York; and Eliel, E. and Wilen, S., "Stereochemistry of organic compounds", John Wiley & Sons, Inc., New York, 1994. The compounds of the present disclosure may contain asymmetric centers or chiral centers, and thus have different stereoisomers. All the stereoisomeric forms of the compounds of the present disclosure include, but in no way limited to, diastereomers, enantiomers, atropisomers, and mixtures thereof, such as racemic mixture, which constitute a part of the present disclosure. Many organic compounds are present in optically active forms, that is, they have the ability to rotate the plane of plane polarized light. When describing optically active compounds, the prefixes *D* and *L* or *R* and *S* are used to represent the absolute configuration of the chiral center of molecule. The prefixes *d* and *l* or (+) and (-) are the symbols used to name the rotation of the plane polarized light of the compound. Specifically, (-) or *l* means that the compound is levorotatory, and the prefix (+) or *d* means that the compound is dextrorotatory. The chemical structures of these stereoisomers are the same, but their stereoscopic structures are different. A specific stereoisomer may be an enantiomer, and a mixture of isomers is usually called an enantiomer mixture. A mixture of 50: 50 enantiomers is called a racemic mixture or racemate, which may result in no stereoselectivity or stereospecificity during the chemical reaction. The terms "racemic mixture" and "racemate" refer to an equimolar mixture of two enantiomers, lacking optical activity.

The term "tautomer" or "tautomeric form" means that structural isomers with different energies can exceed low energy barriers and thus be converted into each other. For example, proton tautomer (i.e., proton-shifted tautomer) includes tautomerism through proton migration, such as keto-enol isomerization and imine-enamine isomerization. The atomic valence (valency) tautomer includes the tautomerism through the recombination of some bonding electrons.

The "hydrate" in the present disclosure refers to a complex formed with water as solvent molecule.

The "solvate" of the present disclosure refers to a complex formed by one or more solvent molecules and the compound of the present disclosure. The solvents for forming solvates include, but are not limited to, water, isopropanol, ethanol, methanol, dimethyl sulfoxide, ethyl acetate, acetic acid, and aminoethanol.

The "ester" in the present disclosure refers to ester that is hydrolysable *in vivo* and formed by the compound of formula (I) containing hydroxyl group. Such esters are, for example, pharmaceutically acceptable esters that can produce parent alcohols by hydrolyzing in humans or animals. The groups of the esters of the compound of formula (I) containing hydroxyl groups that can be hydrolyzed in vivo include, but are not limited to, phosphate group, acetoxymethoxy, 2,2-dimethyl propionyloxymethyl, alkanoyl, benzoyl, phenylacetyl, alkoxycarbonyl, dialkyl carbamoyl, and N-(dialkylaminoethyl)-N-alkyl carbamoyl group, etc.

The "nitrogen oxide" in the present disclosure refers to N-oxide formed by the compound containing several amine functional groups in which one or more nitrogen atoms are oxidized. Specific examples of *N*-oxides are N-oxides of tertiary amines or *N*-oxides of nitrogen atoms in a nitrogen-containing heterocyclic ring. The *N*-oxide can be produced by treating the corresponding amine with an oxidizing agent such as hydrogen peroxide or peracid (e.g., peroxycarboxylic acid) (see Advanced Organic Chemistry, Wiley Interscience, 4-th Edition, Jerry March, pages). In particular, N-oxide can be prepared by a method of L. W. Deady (Syn.Comm.1977, 7, 509-514), for example, reacting an amine compound with m-chloroperoxybenzoic acid (MCPBA) in an inert solvent (such as dichloromethane).

In the specification, the description referring to the term "an embodiment", "an embodiment of the present disclosure", "some embodiments", "an exemplary embodiment", "an example", "a specific example", or "some examples" means that specific features, structures, materials or characteristics described in connection with this embodiment or example are included in at least an embodiment or example of the present disclosure. In this specification, the exemplary expressions of the above terms do not necessarily refer to the same embodiment or example. Moreover, the specific features, structures, materials or characteristics described may be combined in any one or more embodiments or examples in a suitable manner.

Unless otherwise defined, all scientific and technical terms used herein have the same meaning as understood by those skilled in the art. For definitions and terms in the art, the professionals can refer to Current Protocols in Molecular Biology (Ausubel). The abbreviation for amino acid residue is a standard 3-letter and/or 1-letter code used in the art to refer to one of the 20 commonly used L-amino acids.

The luciferase combination provided by the present disclosure includes two luciferases, one of which is Gaussia luciferase, and the other of which is Pleuromamma xiphias luciferase. The Gaussia luciferase, also known as "Gaussian luciferase", is a kind of protein with a molecular weight of 20 kD, and a bioluminescence enzyme derived from the Gaussia princeps of an animal of marine copepods. The receptor molecule of Gaussia luciferase is very small and it catalyzes the oxidation and luminescence of coelenterazine in the presence of oxygen molecules, without the assistance of ATP. The Pleuromamma xiphias luciferase is a copepod luciferase, also known as Daphnia pulex luciferase, which can catalyze the oxidation and luminescence of coelenterazine in the presence of oxygen molecules. The results of the screening of the combinations of luciferases indicate that the combination of luciferases according to the present disclosure can generate stronger fluorescence than other combinations of luciferases, without causing cross-interference.

In the luciferase reporter gene detection system of the present disclosure, the luciferases can be present in the forms of encoding nucleic acids, expression modules, expression vectors, a recombinant strain, an expression product of a recombinant strain or a purified expression product.

Taking the encoding nucleic acid as an example, the detection system described in the present disclosure includes: a mixture of the nucleic acid encoding the Gaussia luciferase and the nucleic acid encoding the Pleuromamma xiphias luciferase; or independently existing nucleic acids encoding the Gaussia luciferase and Pleuromamma xiphas luciferase; or a fusion fragment formed by the nucleic acid encoding the Gaussia luciferase and the nucleic acid encoding the Pleuromamma xiphias luciferase, which is not limited in the present disclosure.

Taking an expression module as an example, the expression module includes a promoter, an encoding nucleic acid, and a terminator. The nucleic acid encoding the Gaussia luciferase and the nucleic acid encoding the Pleuromamma xiphias luciferase are in the same expression module or in two different expression modules. The promoters of the two expression modules where the two encoding nucleic acids are located can be the same or different, which is not limited in the present disclosure.

As an example, the expression vector includes a skeleton and an encoding nucleic acid. The expression vector includes, but is not limited to, a plasmid vector or a virus vector. The nucleic acid encoding the Gaussia luciferase and the nucleic acid encoding the Pleuromamma xiphias luciferase can be expressed by means of the same expression vector, or can be expressed by means of different expression vectors, which is not limited in the present disclosure. When the nucleic acids are expressed by means of different vectors, the skeletons can be the same or different.

The expression strain described in the present disclosure is also referred to as a recombinant host capable of expressing the Gaussia luciferase and/or the Pleuromamma xiphias luciferase. That is, the Gaussia luciferase and the Pleuromamma xiphias luciferase are expressed in the same host. Alternatively, the Gaussia luciferase and the Pleuromamma xiphias luciferase can be expressed in different hosts, which is not limited in the present disclosure. The recombinant host includes a prokaryotic host or a eukaryotic host, which can be constructed by means of electro-transfection, and can also be constructed by means of virus infection. The recombinant host can be *Escherichia coli,* yeast, animal cells or human cells, which is not limited in the present disclosure. The expression of two luciferases in two different hosts may use the same host cell or different host cells.

The expression product or purified expression product of the recombinant strain of the present disclosure is derived from the above-mentioned expression strain or recombinant host. The expression product or purified expression product may be a mixture obtained after the two strains expressing the luciferases, respectively, or may be a product obtained after a strain expressing the two luciferases.

In the present disclosure, "reporter gene", as a molecular biology concept, refers to a gene that can be expressed in cells, tissues/organs or individuals under specific conditions and can produce traits, which are easy to be detected and the experimental materials would not produce, that is, a gene encoding a detectable protein or enzyme. The reporter gene must meet the following conditions in terms of genetic selection and screening detection: 1). it has been cloned and the whole sequence thereof has been determined; 2). the expression product does not exist in the recipient cell, i.e., having no background, and similar endogenous expression product does not exist in the transfected cell; and 3). the expression product can be quantitatively determined. The reporter gene can be used in the manners including, but not limited to: fusing the reporter gene with the gene expression regulation sequence to form a chimeric gene; or fusing the reporter gene with other target genes, and performing nucleic acid expression under the control of the regulation sequence, thereby detecting expression regulation and control of the target genes and investigating the nucleic acid using its expression product.

In the present disclosure, the luciferase reporter gene detection system, also referred to as dual-luciferase luminescence system, includes the luciferase combination and the substrate as described in the present disclosure. The luciferase composition and the substrate exist independently, and emit light after being mixed in a detection system during detection. According to the present disclosure, the substrate is screened, and the obtained luciferase reporter gene detection system can have higher fluorescence intensity, thereby improving the sensitivity of detection.

The detection reagent includes a substance capable of causing the to-be-detected substance to overexpress the Gaussia luciferase and the Pleuromamma xiphias luciferase. The substance capable of causing the to-be-detected substance to overexpress the Gaussia luciferase and the Pleuromamma xiphias luciferase includes, but is not limited to, a plasmid vector or virus vector containing a nucleic acid encoding the Gaussia luciferase and/or a nucleic acid encoding the Pleuromamma xiphias luciferase.

As used herein, the term "polynucleotide" refers to deoxyribonucleic acid (DNA), ribonucleic acid (RNA) or an analogue thereof. The polynucleotides can be single-stranded, double-stranded or can contain both single-stranded and double-stranded sequences. A polynucleotide molecule can be derived from a double-stranded DNA (dsDNA) form (e.g., genomic DNA, PCR and amplification products, etc.); or can be derived from a single-stranded DNA (ssDNA) or RNA and can be converted into a dsDNA form, and vice versa. The exact sequence of the polynucleotide molecules may be known or unknown. The following are illustrative examples of polynucleotides: gene or gene fragment (e.g., probe, primer, EST or SAGE tag), genomic DNA, genomic DNA fragment, exon, intron, messenger RNA (mRNA), transfer RNA, ribosomal RNA, ribozyme, cDNA, recombinant polynucleotide, synthetic polynucleotide, branched polynucleotide, plasmid, vector, isolated DNA of any sequence, isolated RNA of any sequence, nucleic acid probe, primer or amplification copy of any of the above sequences.

The polynucleotides may include nucleotides or nucleotide analogues. The nucleotides usually contain saccharides (such as ribose or deoxyribose), bases and at least one phosphate group. The nucleotides include deoxynucleotides, modified deoxynucleotides, ribonucleotides, modified ribonucleotides, modified phosphate saccharide backbone nucleotides and mixtures thereof. Examples of the nucleotides include, for example, adenosine monophosphate (AMP), adenosine diphosphate (ADP), adenosine triphosphate (ATP), thymidine monophosphate (TMP), thymidine diphosphate (TDP), thymidine triphosphate (TTP), cytidine monophosphate (CMP), cytidine diphosphate (CDP) and cytidine triphosphate (CTP), guanosine monophosphate (GMP), guanosine diphosphate (GDP), guanosine triphosphate (GTP), uridine monophosphate (UMP), uridine diphosphate (UDP), uridine triphosphate (UTP), deoxyadenosine monophosphate (dAMP), deoxyadenosine diphosphate (dADP), deoxyadenosine triphosphate (dATP), deoxythymidine monophosphate (dTMP), deoxythymidine diphosphate (dTDP), deoxythymidine triphosphate (dTTP), deoxycytidine monophosphate (dCMP), deoxycytidine diphosphate (dCDP), deoxycytidine triphosphate (dCTP), deoxyguanosine monophosphate (dGMP), deoxyguanosine diphosphate (dGDP), deoxyguanosine triphosphate (dGTP), deoxyuridine monophosphate (BUMP), deoxyuridine diphosphate (dUDP) and deoxyuridine triphosphate (dUTP).

Nucleotide analogues containing modified bases may also be used in the methods described herein. Exemplary modified bases that can be included in a polynucleotide, whether having a natural backbone or a similar structure, include, for example, inosine, xathanine, hypoxathanine, isocytosine, isoguanine, 2-aminopurine, 5-methylcytosine, 5-hydroxymethylcytosine, 2-aminoaadenine, 6-methyladenine, 6-methylguanine, 2-propylguanine, 2-propyladenine, 2-thiouracil, 2-thiothymine, 2-thiocytosine, 15-halouracil, 15-halocytosine, 5-propynyl uracil, 5-propynyl cytosine, 6-azouracil, 6-azocytosine, 6-azothymine, 5-uracil, 4-thiouracil, 8-haloadenine or guanine, 8-amino adenine or guanine, 8-thioadenine or guanine, 8- thioalkyl adenine or guanine, 8- hydroxy adenine or guanine, 5-halouracil or cytosine, 7-methylguanine, 7-methyladenine, 8-azaguanine, 8-azaadenine, 7-deazaguanine, 7-deazaadenine, 3-deazaguanine, and 3-deoxyadenine, etc.

In general, the nucleotides include nucleotides A, C, G, T or U. As used herein, the term "nucleotide A" refers to a nucleotide containing adenine (A) or a modifier or analogue thereof, such as ATP and dATP. The term "nucleotide G" refers to a nucleotide containing guanine (G) or a modifier or analogue thereof, such as GTP and dGTP. The term "nucleotide C" refers to a nucleotide containing cytosine (C) or a modifier or analogue thereof, such as CTP and dCTP. The term "nucleotide T" refers to a nucleotide containing thymine (T) or a modifier or analogue thereof, such as TTP and dTTP. The term "nucleotide U" refers to a nucleotide containing uracil (U) or a modifier or analogue thereof, such as UTP and dUTP.

### Nucleotide labeling

The present disclosure relates to labeling the nucleotides with different markers individually or in combination, enabling different luciferases to be linked with the nucleotides. As used herein, the molecular marker for labeling a nucleotide and the marker specifically binding thereto can be any molecular pairing capable of specifically binding to each other. The nucleotide and luciferase can be connected to each other by the specific binding between pairing members. Exemplary pairing members include, but are not limited to: (a) haptens or antigenic compounds binding to corresponding antibodies or binding parts or fragments of the corresponding antibodies, such as digoxin-digoxin antibodies, N3G-N3G antibodies, FITC-FITC antibodies; (b) aptamers and protein; (c) non-immune binding pairs (e.g., biotin-avidin, biotin-streptavidin, biotin-neutral antibiotic protein); (d) hormone-hormone binding protein; (e) receptor-receptor agonists or receptor-receptor antagonists; (f) homoagglutinin-carbohydrate; (g) enzyme-enzyme cofactor; and (h) enzyme-enzyme inhibitor.

In a specific embodiment, the first molecular marker and the second molecular marker are small molecular markers, which are selected from biotin, digoxin, N3G, or FITC. The two luciferases can specifically bind to the first molecular marker and the second molecular marker, respectively. For example, in a specific embodiment, if the first molecular marker is biotin, the first luciferase can be luciferase labeled with streptavidin; and if the second molecular marker is digoxin, the second luciferase may be luciferase labeled with digoxin antibody, which is different from the first luciferase. Preferably, the first luciferase is the Gaussia luciferase as described in the present disclosure or a mutant thereof, and the second luciferase is the Pleuromamma xiphias luciferase as described in the present disclosure or a mutant thereof.

As used herein, the expression "a first compound is linked with a first molecular marker" means that all the first compounds are linked with the first molecular marker, or some of the first compounds are linked with the first molecular marker but the remaining first compounds are linked with no molecular marker. Similarly, the expression "a second compound is linked with a second molecular marker" means that all the second compounds are linked with the second molecular marker, or some of the second compounds are linked with the second molecular marker but the remaining second compounds are linked with no molecular marker. The expression "a third compound is linked with a first molecular marker and a second molecular marker" means that all the third compounds are linked with the first molecular marker and the second molecular marker, or some of the third compounds are linked with the first molecular marker and the second molecular marker, but the remaining third compounds are linked with no molecular marker.

### Sequencing of polynucleotides

Preferably, the dual-luciferase reporter gene detection system of the present disclosure is suitable for synthetic sequencing. The synthetic sequencing as used herein may be one of various synthetic sequencing methods well known in the related art. Basically, the synthetic sequencing includes: first hybridizing a to-be-sequenced nucleic acid molecule with a sequencing primer, and then performing polymerization of a nucleotide or nucleotide analogue as described herein at the 3' end of the sequencing primer by using the sequenced nucleic acid molecule as a template in the presence of polymerase. After the polymerization, the type of the nucleotide molecule is identified by detecting the fluorescence signal emitted by luciferase. After luciferase is removed from the labeled nucleotides, the next polymerization sequencing cycle is carried out.

A method for determining a sequence of a target polynucleotide can be carried out by: denaturing the target polynucleotide sequence, contacting the target polynucleotide with different nucleotides, to form a complement of the target nucleotide, and detecting the incorporation of the nucleotides. The method utilizes polymerization, so that the polymerase can extend the complementary chain by incorporating the correct nucleotides complementary to the target. The polymerization reaction also requires special primers to initiate the polymerization.

For each cycle of reaction, the nucleotides are incorporated by means of polymerase, and then the incorporation event is determined. There are many different polymerases, and those skilled in the art can easily determine the most suitable polymerase. The preferred enzymes include DNA polymerase I, Klenow fragment, DNA polymerase III, T4 or T7 DNA polymerase, Taq polymerase or Vent polymerase. The polymerase engineered to have specific properties can also be used.

The sequencing method is preferably performed on target polynucleotides arranged on a solid support. A plurality of target polynucleotides may be immobilized on the solid support through linker molecules, or may be attached to particles such as microspheres, which may also be attached to a solid support material.

The polynucleotides may be attached to the solid support by various methods, including the use of biotin-streptavidin interaction. The methods for immobilizing the polynucleotides on a solid support are well known in the related art, and include lithographic techniques and spotting each polynucleotide at a specific site on the solid support. Suitable solid supports are well known in the art and include glass slides and beads, ceramic and silicon surfaces and plastic materials.

The support is usually planar. Although microbeads (microspheres) can also be used as the support, and the microbeads may be further attached to another solid support by known methods. The microspheres can have any suitable size, with a diameter ranging from 10 to 100 nanometers. In a preferred embodiment, the polynucleotide is attached directly to a flat surface, preferably to a flat glass surface. The connection is preferably carried out in the form of covalent bonds. The array used is preferably a monomolecular array, which includes the polynucleotides located in unique optically distinguishable regions, such as described in international application number WO00/06770.

The necessary conditions for polymerization are well known to those skilled in the art. In order to carry out the polymerase reaction, the primer sequence is necessarily annealed to the target polynucleotide first, and the primer sequence is identified by the polymerase and serves as the starting site for the subsequent extension of the complementary strand. The primer sequence may be added as a separate component with respect to the target polynucleotide. In addition, the primer and the target polynucleotide can be part of a single-stranded molecule, respectively, and the primer portion and a portion of the target form an intramolecular duplex, that is, a hairpin ring structure. The structure can be immobilized on the solid support at any site of the molecule. Other conditions necessary for carrying out the polymerase reaction are well known to those skilled in the art, including the temperature, pH and buffer composition.

Subsequently, the labeled nucleotide or nucleotide analogue of the present disclosure is contacted with the target polynucleotide to carry out the polymerization. The nucleotides or nucleotide analogues can be added sequentially, that is, the respective types of nucleotides (A, C, G or T/U) can be added separately, or several types of nucleotides (A, C, G or T/U) can be added at the same time.

The polymerization step is performed for a duration, which is sufficient to incorporate one nucleotide.

The unincorporated nucleotides are then removed, for example, by removing the solution phase of the reaction system in the previous step, leaving the duplex attached to the support.

Subsequently, the two luciferases can be added for binding reaction, and the two luciferases can specifically bind to the molecular markers on nucleotides, thereby connecting the luciferases to the incorporated nucleotides. Then, the incorporated nucleotides can be identified by adding the corresponding luciferase substrates and detecting the fluorescence signal.

In a specific embodiment, four deoxynucleotide analogues are labeled with different small molecular markers, i.e., biotin (B for short ) and digoxin (D for short). For example, nucleotide A is labeled with B, nucleotide C is labeled with B and D, nucleotide T is labeled with D, and nucleotide G is not labeled. The hydroxyl group at the 3'-end of the four deoxynucleotide analogues labeled with different small molecules is blocked to ensure that only one deoxynucleotide is connected in each sequencing reaction. During the sequencing reaction, the mixture of the four labeled deoxynucleotide analogues and sequencing polymerase is introduced, and under the action of polymerase, one deoxynucleotide analogue is incorporated into the 3' end of the growing nucleic acid chain based on the principle of base complementary pairing. The unbound deoxynucleotide analogues can be removed by removing the solution phase of the reaction system in the previous step and leaving the duplex attached to the support. Then, two different luciferases are added. The first luciferase is labeled with streptavidin, which binds to nucleotide A or nucleotide C labeled with small molecule B, and the second luciferase is labeled with digoxin antibody, which binds to nucleotide C or nucleotide T labeled with small molecule D. After the unbound luciferase is removed using elution buffer, the substrate of the first luciferase is added, and the nucleotide linked with the first luciferase emits light, and the signal is detected by a detector; and when the substrate of the second luciferase is added, the nucleotide linked with the second luciferase emits light, and the signal is detected by a detector, thereby obtaining the luminescence situation listed in the following table. In this way, the base can be identified (1 indicates that the fluorescence signal is detected, and 0 indicates that the fluorescence signal is not detected).

| | First detection | Second detection |
|---|---|---|
| A | 1 | 0 |
| C | 1 | 1 |
| G | 0 | 0 |
| T | 0 | 1 |

In a specific embodiment, the connection of two different luciferases (the Gaussia luciferase or mutant thereof, and the Pleuromamma xiphias luciferase or mutant thereof) with the labeled nucleotides and the signal detection can be carried out separately. The first luciferase labeled by streptavidin is first added, and linked with nucleotide A or nucleotide C labeled with small molecule B. After the unbound first luciferase is removed with elution buffer, the substrate of the first luciferase is added, and the nucleotide linked with the first luciferase emits light, and the signal is detected by a detector. After removing the reaction solution, the second luciferase labeled with digoxigenin antibody is added and binds to nucleotide C or nucleotide T labeled with small molecule D, then the unbound second luciferase is removed with elution buffer, the substrate of the second luciferase is added, and the base linked with the second luciferase emits light, and the signal is detected by a detector. Thus, the luminescence situation shown in the above table is obtained, and the base can be identified.

### Detection of fluorescence signal

The methods of detecting fluorescence signals are well known in the art. For example, the fluorescence signals can be detected by a device for detecting wavelength of fluorescence. Such a device is well known in the related art. For example, such a device may be a confocal scanning microscope, which can scan a surface of a solid support with a laser to image the fluorophores directly bound to nucleic acid molecules to be sequenced. In addition, each of the resulting signals can be observed, for example, with a sensitive 2-D detector such as a charge coupled detector (CCD). Other techniques such as scanning near-field optical microscopy (SNOM) can also be used.

The reagents or instruments used in the present disclosure can be purchased from the market. The Gaussia luciferase (GenBank: AY015993.1) used in the example was prepared from the *E.coli* expression system, and the expression vector was pET28a. The Pleuromamma xiphias luciferase (PMID: 23886588) was also prepared from *E.coli* expression system, and the expression vector was pET28a.

The present disclosure will be further illustrated below in conjunction with the examples.

### Example 1 Pairing luminescence detection of dual-luciferase system (wild type)

Experimental purpose: screening out a luminous combination with high brightness and low cross-interference by testing luminous intensity of different substrates to wild-type Gaussia luciferase and wild-type Pleuromamma xiphias luciferase.

### Experimental steps:

(1) Wild-type Gaussia luciferase (WT-Gluc) has the amino acid sequence as shown below.

KPTENNEDFNIVAVASNFATTDLDADRGKLPGKKLPLEVLKEMEANARKA
GCTRGCLICLSHIKCTPKMKKFIPGRCHTYEGDKESAQGGIGEAIVDIPEIPGFKDLEP
MEQFIAQVDLCVDCTTGCLKGLANVQCSDLLKKWLPQRCATFASKIQGQVDKIKGA
GGD (SEQ ID NO: 1).

Wild-type Gaussia luciferase (WT-Gluc) has the nucleotide sequence as shown below.

AAACCAACTGAAAACAATGAAGATTTCAACATTGTAGCTGTAGCTAGCA
ACTTTGCTACAACGGATCTCGATGCTGACCGTGGTAAATTGCCCGGAAAAAAATTA
CCACTTGAGGTACTCAAAGAAATGGAAGCCAATGCTAGGAAAGCTGGCTGCACTA
GGGGATGTCTGATATGCCTGTCACACATCAAGTGTACACCCAAAATGAAGAAGTTT
ATCCCAGGAAGATGCCACACCTATGAAGGAGACAAAGAAAGTGCACAGGGAGGA

ATAGGAGAGGCTATTGTTGACATTCCTGAAATTCCTGGGTTTAAGGATTTGGAACC
CATGGAACAATTCATTGCACAAGTTGACCTATGTGTAGACTGCACAACTGGATGCC
TCAAAGGTCTTGCCAATGTGCAATGTTCTGATTTACTCAAGAAATGGCTGCCACAA
AGATGTGCAACTTTTGCTAGCAAAATTCAAGGCCAAGTGGACAAAATAAAGGGTG
CCGGTGGTGAT (SEQ ID NO: 2).

Wild-type Pleuromamma xiphias luciferase (WT-Pxluc) has the amino acid sequence as shown below.

QPTENKQESHIVDSDLDGDRGRKLPGKKLPIEVLKIMEANARRAGCTRGC
LICLSKIKCTAKMKRYIPGRCHTYEGDKSIGQGGIGGPIVDIPEIIGFKNMEPMDQFIAQ
VDLCADCTTGCLKGLANVRCNDLLKKWLPDRCAGFADKIQNEVDSIKGMAGDR
(SEQ ID NO: 3).

Wild-type Pleuromamma xiphias luciferase (WT-Pxluc) with signal peptide has the nucleotide sequence as shown below.

MYIKVWFGLACLSLVLAOPTENKOESHIVDSDLDGDRGRKLPGKKLPIEVL
KIMEANARRAGCTRGCLICLSKIKCTAKMKRYIPGRCHTYEGDKSIGOGGIGGPIVDIP
EIIGFKNMEPMDOFIAOVDLCADCTTGCLKGLANVRCNDLLKKWLPDRCAGFADKIQ
NEVDSIKGMAGDR (SEQ ID NO: 4).

Wild-type Pleuromamma xiphias luciferase (WT-Pxluc) has the nucleotide sequence as shown below.

CAACCAACTGAAAACAAGCAGGAGTCTCATATTGTAGATTCAGATCTTG
ATGGCGACCGTGGTAGGAAGTTGCCCGGAAAAAAGCTTCCTATAGAAGTACTCAA
AATCATGGAAGCCAATGCCAGGAGAGCTGGTTGCACTAGAGGATGTCTCATATGTC
TTTCAAAAATCAAGTGTACAGCCAAAATGAAGCGATACATTCCAGGGAGATGTCAT
ACTTATGAAGGAGATAAATCTATTGGACAGGGAGGCATAGGTGGCCCTATTGTTGAT
ATTCCTGAAATTATTGGATTCAAGAACATGGAACCCATGGATCAGTTCATCGCACA
AGTTGATCTGTGCGCCGACTGTACAACTGGGTGCCTGAAAGGCCTTGCTAATGTTA
GGTGCAATGACTTGCTGAAGAAATGGCTGCCTGACAGATGTGCTGGTTTTGCCGA
CAAAATTCAAAATGAAGTGGATAGTATCAAGGGCATGGCTGGGGATCGC (SEQ ID
NO: 5).

(2) Coelenterazine derivatives were used as substrates: the substrates f-CTZ, N0, N1, N3, N4, N5, N6, N7 and N8 were synthesized according to the method disclosed in patent application PCT/CN2018/107646.

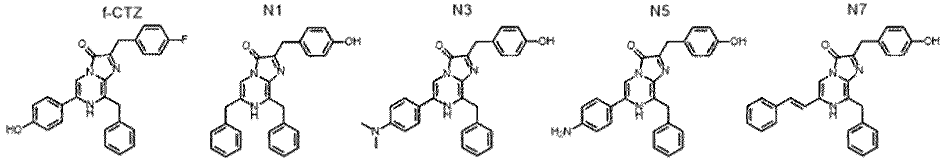

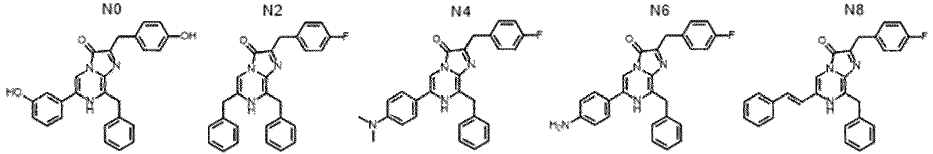

(3) According to the conventional methods, such as the method disclosed in PCT/CN2017/082180, an expression vector (plasmid) containing the wild-type Gaussia luciferase gene and the wild-type Pleuromamma xiphias luciferase gene was constructed. The plasmid was transformed into *Escherichia coli* OrigamiB (DE3), which was coated on a plate, and a single colony was picked from the plate, cultured overnight at 37°C, diluted at a ratio of 1:100 on the next day, transferred to 3 mL of fresh LB medium containing ampicillin resistance (100 µg/mL), cultured with shaking at 37°C and 200 rpm for 4 hours, and cooled on ice for 1 hour. The inducer IPTG was added at a final concentration of 1 mM and the induction was preformed overnight at 16°C.

On the next day, the bacteria were collected by centrifugation, resuspended with 500 µL lysate (50 mM Tris-HCl pH 8.0, 500 mM NaCl, 0.2% lysozyme, PMSF), lysed for 20 minutes at room temperature, frozen with liquid nitrogen and thawed for 3 times, and centrifuged for 30 minutes at 18,000 g. The supernatant was taken, which was purified by using a centrifugal Ni column.

The protein eluted by Ni column was dialyzed with dialysis buffer (25 mM Tris, pH 8.0, 250 mM NaCl) at 4°C overnight. The concentration of luciferase was accurately determined by using a BCA quantitative kit (Thermoscientific^{™} Pierce^{™} BCA protein assay kit).

The two luciferases were diluted to a concentration of 1 µg/mL with a diluent (50 mM Tris-HCl pH 8.0, 100 mM NaCl, 0.1%(v/v) Tween-20), and 10 µL of each luciferase was taken and added to a black 96-well plate. The substrate was diluted to 100 µM with the same diluent, 90 µL of the substrate was taken add into the 96-well plate containing the above luciferases (90 µL/well), and the luminescence intensity was read by using a light-emitting module of a microplate reader.

The test data of the Gaussia luciferase and the Pleuromamma xiphias luciferase on different substrates were shown in Table 1, in which the relative intensity is a percentage relative to the activity of wild-type Pleuromamma xiphias luciferase and compound N0. The results indicated that: the Gaussia luciferase had the highest fluorescence intensity in incorporation with the substrate f-CTZ, and the Pleuromamma xiphias luciferase had the highest fluorescence intensity in incorporation with the substrate N0. That is, in this dual-luciferase reporter gene detection system, the optimal luminescent combination with high fluorescence brightness and low cross-interference obtained after screening is using f-CTZ to detect the Gaussia luciferase, and using N0 to detect the Pleuromamma xiphias luciferase.

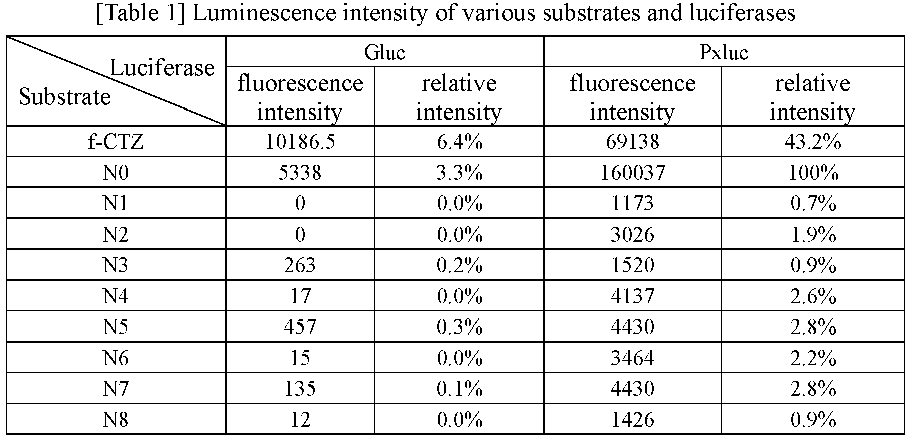

### Example 2 Pairing luminescence detection of dual-luciferase system (mutant-type)

Experimental purpose: screening out a luminous combination with high brightness and low cross-interference by testing the luminous intensity of different substrates to mutant-type Gaussia luciferase and mutant-type Pleuromamma xiphias luciferase.

### Experimental steps:

### (1) Mutant-type Gaussia luciferase E1-A3, G2-F11 and G2-F8:

a) E1-A3: based on SEQ ID NO: 1, the following sites were mutated: F26R, V29F, A32V, S33E, A36V, L40I, K66P, H79K, P84L, E102S, S103T, A104G, E110P, L124M, and V138E.

The mutant E1-A3 has the nucleotide sequence as shown below.

ATGAAACCAACTGAAAACAATGAAGATCGCAACATTTTCGCTGTAGTTG
AGAACTTTGTTACGACGGATATTGATGCTGACCGTGGTAAATTGCCCGGAAAAAAA
TTACCACTTGAGGTACTCAAAGAAATGGAAGCCAATGCTAGGCCTGCTGGCTGCA
CTAGGGGATGTCTGATATGCCTGTCAAAAATCAAGTGTACACTCAAAATGAAGAAG
TTTATCCCAGGAAGATGCCACACCTATGAAGGAGACAAATCAACTGGACAGGGAG
GAATAGGACCGGCTATTGTTGACATTCCTGAAATTCCTGGGTTTAAGGATATGGAAC
CCATGGAACAATTCATTGCACAAGTTGACCTATGTGAAGACTGCACAACTGGATGC
CTCAAAGGTCTTGCCAATGTGCAATGTTCTGATTTACTCAAGAAATGGCTGCCACA
AAGATGTGCAACTTTTGCTAGCAAAATTCAAGGCCAAGTGGACAAAATAAAGGGT
GCCGGTGGTGAT (SEQ ID NO: 6).

b) G2-F11: based on SEQ ID NO: 1, the following sites were mutated: E24K, H79K, P84L, E102S, S103T, A104G, E110P, L124G, Q152R, Q163D, S170N, Q175E, K178T, A182M, G183N), G2-E1(H79K, P84L, E102S, S103T, A104G, E110P, L124I, Q152H, Q163D, T167S, S170T, G174K, A182M, and G183A.

The mutant G2-F11 has the nucleotide sequence as shown below.

ATGAAACCAACTGAAAACAATAAAGATTTCAACATTGTAGCTGTAGCTA
GCAACTTTGCTACAACGGATCTCGATGCTGACCGTGGTAAATTGCCCGGAAAAAAA
TTACCACTTGAGGTACTCAAAGAAATGGAAGCCAATGCTAGGAAAGCTGGCTGCA
CTAGGGGATGTCTGATATGCCTGTCAAAAATCAAGTGTACACTCAAAATGAAGAAG
TTTATCCCAGGAAGATGCCACACCTATGAAGGAGACAAATCAACTGGACAGGGAG
GAATAGGACCGGCTATTGTTGACATTCCTGAAATTCCTGGGTTTAAGGATGGGGAA
CCCATGGAACAATTCATTGCACAAGTTGACCTATGTGTAGACTGCACAACTGGATG
CCTCAAAGGTCTTGCCAATGTGCGATGTTCTGATTTACTCAAGAAATGGCTGCCAG
ACAGATGTGCAACTTTTGCTAACAAAATTCAAGGCGAAGTGGACACAATAAAGGG
TATGAACGGTGAT (SEQ ID NO: 7).

c) G2-F8: based on SEQ ID NO: 1, the following sites were mutated: H79K, P84L, E102S, S103T, A104G, E110P, L124M, Q152H, Q163D, T167S, S170T, G174K, A182M, and G183A.

The mutant G2-F8 has the nucleotide sequence as shown below.

ATGAAACCAACTGAAAACAATGAAGATTTCAACATTGTAGCTGTAGCTA
GCAACTTTGCTACAACGGATCTCGATGCTGACCGTGGTAAATTGCCCGGAAAAAAA
TTACCACTTGAGGTACTCAAAGAAATGGAAGCCAATGCTAGGAAAGCTGGCTGCA
CTAGGGGATGTCTGATATGCCTGTCAAAAATCAAGTGTACACTCAAAATGAAGAAG
TTTATCCCAGGAAGATGCCACACCTATGAAGGAGACAAATCAACTGGACAGGGAG
GAATAGGACCGGCTATTGTTGACATTCCTGAAATTCCTGGGTTTAAGGATATGGAAC
CCATGGAACAATTCATTGCACAAGTTGACCTATGTGTAGACTGCACAACTGGATGC
CTCAAAGGTCTTGCCAATGTGCATTGTTCTGATTTACTCAAGAAATGGCTGCCAGA
CAGATGTGCAAGTTTTGCTACCAAAATTCAAAAGCAAGTGGACAAAATAAAGGGT
ATGGCTGGTGAT (SEQ ID NO: 8).

Mutant-type Pleuromamma xiphias luciferase P26-95:
d) P26-95: based on SEQ ID NO: 3, the following sites were mutated: G83A, and G84P.

The mutant P26-95 has the nucleotide sequence as shown below.

CAACCAACTGAAAACAAGCAGGAGTCTCATATTGTAGATTCAGATCTTG
ATGGCGACCGTGGTAGGAAGTTGCCCGGAAAAAAGCTTCCTATAGAAGTACTCAA
AATCATGGAAGCCAATGCCAGGAGAGCTGGTTGCACTAGAGGATGTCTCATATGTC
TTTCAAAAATCAAGTGTACAGCCAAAATGAAGCGATACATTCCAGGGAGATGTCAT
ACTTATGAAGGAGATAAATCTATTGGACAGGCACCGATAGGTGGCCCTATTGTTGAT
ATTCCTGAAATTATTGGATTCAAGAACATGGAACCCATGGATCAGTTCATCGCACA
AGTTGATCTGTGCGCCGACTGTACAACTGGGTGCCTGAAAGGCCTTGCTAATGTTA
GGTGCAATGACTTGCTGAAGAAATGGCTGCCTGACAGATGTGCTGGTTTTGCCGA
CAAAATTCAAAATGAAGTGGATAGTATCAAGGGCATGGCTGGGGATCGC (SEQ ID
NO: 9).

According to the method as described in Example 1, an expression vector containing the gene of the Gaussia luciferase mutant and the gene of the Pleuromamma xiphias luciferase mutant was constructed, a recombinant strain was constructed, the mutant-type luciferases were expressed, and the luminous intensity of substrates and luciferases was detected.

The results indicated that the Gaussia luciferase mutants E1-A3, G2-F11 and G2-F8 had the highest fluorescence intensity in cooperation with the substrate f-CTZ, and the Pleuromamma xiphias luciferase mutant P26-95 had the highest fluorescence intensity in cooperation with the substrate N0. That is, in this dual-luciferase reporter gene detection system, the optimal luminescent combination with high fluorescence brightness and low cross interference obtained after screening is using the substrate f-CTZ to detect the Gaussia luciferase mutant E1-A3, G2-F11 or G2-F8, and using the substrate N0 to detect Pleuromamma xiphias luciferase mutant P26-95.

### Example 3 Use of dual-luciferase reporter gene detection system

The expression plasmid having the wild-type Gaussia luciferase (WT-Gluc) gene and the wild-type Pleuromamma xiphias luciferase (WT-Pxluc) gene as reporter genes was constructed, transfected into a 24-well, which was plated with 0.5×10⁴ HEK293 cells in advance, placed in a cell incubator, and cultured overnight at 5% CO₂ and 37°C.

On the next day, the culture solution in the culture plate/dish was discarded, enough PBS was added to gently wash the cells. The PBS washing liquid was completely discarded. 50 µL of 1 x Passive lysis buffer (Promega) was added to lyse the cells. The cell suspension was transferred into a 1.5 mL centrifuge tube, and fully suspended and oscillated on a vortex shaker for 30 seconds. The lysed cell suspension was used for luminescence assay, or centrifuged at 100 g for 30 seconds and then the supernatant was taken for luminescence assay.

50 µL of cell suspension or supernatant was added to a black 96-well plate. The coelenterazine f-CTZ was diluted with a diluent (50 mM Tris-HCl pH 8.0, 100 mM NaCl, 0.1%(v/v) Tween-20) to 100 µM, 50 µL of the diluted coelenterazine f-CTZ was taken and added into the above 96-well plate (50 µL/well) containing cell suspension or supernatant, and the luminous intensity was read by using a light-emitting module of a microplate reader. After the assay, 1x stop&glo reagent (Promega) was added to terminate the luminescence of f-CTZ, and the N0 substrate, which had been diluted to 100 µM with the same diluent, was added in the same system, and the luminescence intensity was read by the light-emitting module of the microplate reader.

The results indicated that the dual-luciferase reporter gene detection system can be used to detect the luciferases expressed in cells.

The above is only the preferred embodiment of the present disclosure. It should be pointed out that those skilled in the art can make several modifications and improvements without departing from the principle of the present disclosure, and these modifications and improvements should also be regarded as the protection scope of the present disclosure.

## Claims

1. A dual-luciferase reporter gene detection system, comprising:
Gaussia luciferase;
Pleuromamma xiphias luciferase; and
a substrate, wherein the substrate is a compound represented by formula (I), or a stereoisomer, geometric isomer, tautomer, salt, nitrogen oxide, hydrate or solvate of the compound represented by formula (I): 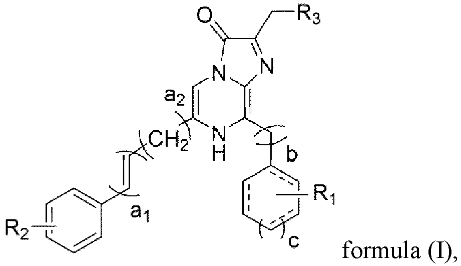
wherein:
R₁ and R₂ are each independently selected from H, D, F, Cl, Br, I, OH, NH₂, NO₂, CN, N₃, C₁₋₆ alkyl, C₂₋₆ alkenyl, C₂₋₆ alkynyl, halogenated C₁₋₆ alkyl, C₁₋₆ alkoxy, C₁₋₆ hydroxyalkyl, or C₁₋₆ alkylamino;
R₃ is an optionally substituted aryl, heterocyclic group or heteroaryl;
a₁ is 0 or any integer ranging from 1 to 6;
a₂ is 0 or any integer ranging from 1 to 6;
b is 0 or 1; and
c is 0, 1 or 2.

2. The dual-luciferase reporter gene detection system according to claim 1, wherein the Gaussia luciferase is any one of I) to III):
I) Gaussia luciferase having an amino acid sequence as set forth in SEQ ID NO: 1;
II) luciferase having at least 85% homology with the amino acid sequence of the Gaussia luciferase as defined in I) and having the same or similar function as the Gaussia luciferase as defined in I);
III) luciferase having the amino acid sequence of the Gaussia luciferase as defined in I) in which one or more amino acid residues are modified, substituted, deleted or added and having Gaussia luciferase activity.

3. The dual-luciferase reporter gene detection system according to claim 2, wherein the Gaussia luciferase is an optimized mutant and has a mutation at least one of following mutation sites in the amino acid sequence as set forth in SEQ ID NO: 1:
sites 24, 26, 27, 29, 30, 31, 32, 33, 36, 37, 40, 66, 79, 84, 88, 102, 103, 104, 110, 123, 124, 138, 152, 163, 167, 170, 174, 175, 178, 182, and 183.

4. The dual-luciferase reporter gene detection system according to claim 3, wherein the Gaussia luciferase mutant has at least one of the following mutations:
1) E at site 24 being mutated to K;
2) F at site 26 being mutated to R or L;
3) N at site 27 being mutated to D;
4) V at site 29 being mutated to F or L;
5) A at site 30 being mutated to G or D;
6) V at site 31 being mutated to I;
7) A at site 32 being mutated to V;
8) S at site 33 being mutated to E, R, or K;
9) A at site 36 being mutated to V or I;
10) T at site 37 being mutated to N or E;
11) L at site 40 being mutated to I or T;
12) K at site 66 being mutated to P, S, I, R, or N;
13) H at site 79 being mutated to K;
14) P at site 84 being mutated to A, L, K, or V;
15) K at site 88 being mutated to R;
16) E at site 102 being mutated to D, A, S, K, or N;
17) S at site 103 being mutated to T;
18) A at site 104 being mutated to G;
19) E at site 110 being mutated to P, G, or A;
20) D at site 123 being mutated to N;
21) L at site 124 being mutated to M, G, or I;
22) V at site 138 being mutated to E or D;
23) Q at site 152 being mutated to R or H;
24) Q at site 163 being mutated to D;
25) S at site 170 being mutated to N or T;
26) G at site 174 being mutated to K;
27) Q at site 175 being mutated to E;
28) K at site 178 being mutated to T;
29) A at site 182 being mutated to M;
30) G at site 183 being mutated to N or A.

5. The dual-luciferase reporter gene detection system according to claim 1, wherein the Pleuromamma xiphias luciferase is any one of I) to III):
I) Pleuromamma xiphias luciferase having an amino acid sequence as set forth in SEQ ID NO: 3;
II) luciferase having at least 85% homology with the amino acid sequence of the Pleuromamma xiphias luciferase as defined in I) and having the same or similar function as the Pleuromamma xiphias luciferase as defined in I);
III) luciferase having the amino acid sequence of the Pleuromamma xiphias luciferase as defined in I) in which one or more amino acid residues are modified, substituted, deleted or added and having Pleuromamma xiphias luciferase activity.

6. The dual-luciferase reporter gene detection system according to claim 5, wherein the Pleuromamma xiphias luciferase is an optimized mutant and has a mutation at least one of following mutation sites in the amino acid sequence as set forth in SEQ ID NO: 3:
sites 81, 82, 83, and 84.

7. The dual-luciferase reporter gene detection system according to claim 6, wherein the Pleuromamma xiphias luciferase mutant has at least one of the following mutations:
(1) G at site 81 being mutated to L, P, Q, S, or T;
(2) Q at site 82 being mutated to R, W, I, Y, A, L, F, V, P, E, or M;
(3) G at site 83 being mutated to S, Q, R, W, T, A, or L;
(4) G at site 84 being mutated to F, R, S, C, Y, L, I, K, V, or P.

8. The dual-luciferase reporter gene detection system according to claim 1, wherein the compound represented by formula (I) further has at least one of the following additional technical features:
R₁ is H;
R₂ is H, NH₂, OH, or C₁₋₆ alkylamino, and preferably H, NH₂, 3-OH, 4-OH, or dimethylamino;
R₃ is a substituted aryl, preferably OH-C₆H₅ or F-C₆H₅, and more preferably 4-OH-C₆H₅ or 4-F-C₆H₅;
a₁ is 0 or 1;
a₂ is 0 or 1;
preferably, when a₁ is 0, a₂ is 1; when a₁ is 1, a₂ is 0; or when a₁ is 0, a₂ is 0;
b is 1; and
c is 1.

9. The dual-luciferase reporter gene detection system according to claim 8, wherein the compound represented by formula (I) comprises any two of the following compounds: 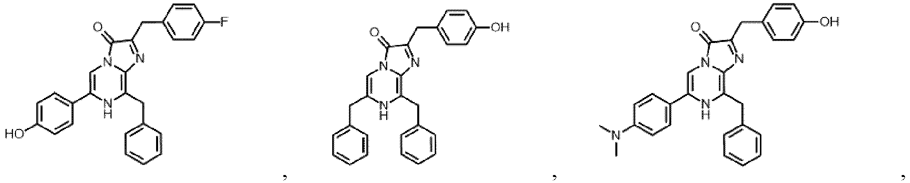 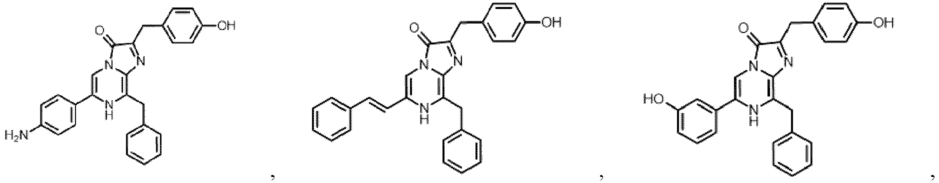 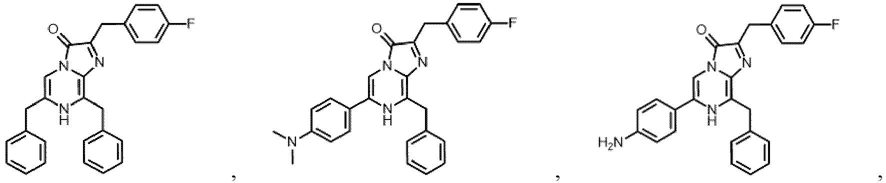 or

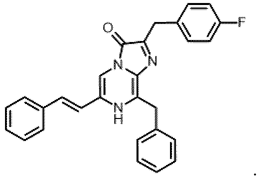

10. The dual-luciferase reporter gene detection system according to claim 9, wherein:
a substrate of the Gaussia luciferase is: 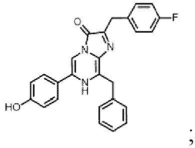 and
a substrate of the Pleuromamma xiphias luciferase is

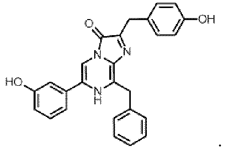

11. The dual-luciferase reporter gene detection system according to claim 1, wherein:
a molar ratio of the Gaussia luciferase to the Pleuromamma xiphias luciferase is 1: (0.01 to 100);
a molar ratio of the Gaussia luciferase to the substrate is 1: (1 to 1000);
a molar ratio of the Pleuromamma xiphias luciferase to the substrate is 1: (1 to 1000).

12. Use of the dual-luciferase reporter gene detection system according to any one of claims 1 to 11 in the preparation of a detection reagent.

13. The use according to claim 12, wherein the detection reagent comprises a cell imaging reagent, an amino acid labeling reagent, a protein labeling and localization reagent, an antibody specific recognition reagent, a nucleic acid labeling reagent, or a gene sequencing reagent.

14. A fluorescence detection kit, comprising the dual-luciferase reporter gene detection system according to any one of claims 1 to 11.

15. The fluorescence detection kit according to claim 14, further comprising a reaction buffer, the reaction buffer comprising water, Tris-HCl, NaCl, and Tween-20.

16. A method for detecting a luminescent signal of luciferase, comprising:
contacting the substrate as defined in any one of claims 1 to 11 with a sample, the sample comprising the Gaussia luciferase and the Pleuromamma xiphias luciferase as defined in any one of claims 1 to 11; and
detecting luminescent signals generated by the Gaussia luciferase and the Pleuromamma xiphias luciferase.

17. The method according to claim 16, wherein the sample is a cell, a culture containing a cell, or a lysate of a cell, the cell expressing the Gaussia luciferase and the Pleuromamma xiphias luciferase as defined in any one of claims 1 to 11.

18. The method according to claim 16, wherein the sample is a nucleotide analogue capable of specifically binding to the Gaussia luciferase and the Pleuromamma xiphias luciferase as defined in any one of claims 1 to 11, the nucleotide analogue specifically binding to the luciferase through a nucleophile.

19. The method according to claim 18, wherein the nucleophile is an antigen-antibody complex, or a biotin-streptavidin or a digoxin-digoxin antibody.

20. The method according to claim 18 or 19, wherein the nucleotide analogue consists of nucleotide molecules containing four different types of bases; wherein:
a first nucleotide molecule is capable of specifically binding to the Gaussia luciferase;
a second nucleotide molecule is capable of specifically binding to the Pleuromamma xiphias luciferase;
a third nucleotide molecule is capable of specifically binding to the Gaussia luciferase and the Pleuromamma xiphias luciferase; and
a fourth nucleotide molecule neither binds to the Gaussia luciferase nor the Pleuromamma xiphias luciferase.

21. The method according to any one of claims 16 to 20, further comprising: determining a type of the nucleotide molecule based on the luminescence signal.

22. A method for sequencing a nucleic acid molecule, comprising:
(1) providing a to-be-sequenced nucleic acid molecule connected to a support, or connecting the to-be-sequenced nucleic acid molecule onto a support;
(2) forming a reaction system containing a solution phase and a solid phase by adding a primer for initiating a nucleotide polymerization reaction, a polymerase for performing the nucleotide polymerization reaction, and four compounds, wherein the four compounds are derivatives of nucleotides A, (T/U), C and G, respectively and have an ability of base complementary pairing, and wherein a hydroxyl group (-OH) at 3' site of ribose or deoxyribose of the four compounds is protected by a protecting group,
a first compound is linked with a first molecular marker,
a second compound is linked with a second molecular marker,
a third compound is linked with the first molecular marker and the second molecular marker, or a part of the third compound is linked with the first molecular marker and the other part of the third compound is linked with the second molecular marker, and
a fourth compound is not linked with a molecular marker;
(3) annealing the primer onto the to-be-sequenced nucleic acid molecule, wherein the primer, as an initial growing nucleic acid chain, forms a duplex connected to the support together with the to-be-sequenced nucleic acid molecule;
(4) performing the nucleotide polymerization reaction using the polymerase under the conditions of allowing the polymerase to perform a nucleotide polymerization reaction, to incorporate one of the four compounds to the 3' end of the growing nucleic acid chain;
(5) contacting the duplex in the previous step with the Gaussia luciferase and the Pleuromamma xiphias luciferase as defined in any one of claims 1 to 11 for binding reaction, wherein the Gaussia luciferase and the Pleuromamma xiphias luciferase are capable of specifically binding to the first molecular marker and the second molecular marker, respectively, enabling the luciferases to undergo fluorescence reaction in the presence of a substrate, and detecting emitted fluorescence signal;
(6) removing the protective groups and molecular markers of the incorporated nucleotides;
(7) optionally, repeating steps (4) to (6) or steps (3) to (6) for one or more times, to obtain the sequence information of the nucleic acid molecule.
